# EUROPÄISCHE PATENTSCHRIFT

(11) **EP 2 077 976 B1**
(45) Veröffentlichungstag und Bekanntmachung des Hinweises auf die Patenterteilung: **18.05.2016**
(21) Anmeldenummer: 07818644.2
(22) Anmeldetag: 02.10.2007
(51) Int. Cl.: C02F 1/50

(54) **BIOAKTIVE, RUTHENIUMHALTIGE BESCHICHTUNG UND VORRICHTUNG**
BIOACTIVE, RUTHENIUM-CONTAINING COATING AND DEVICE
REVÊTEMENT BIOACTIF CONTENANT DU RUTHÉNIUM, ET DISPOSITIF CORRESPONDANT

(30) Priorität: 13.10.2006 DE 102006049108
(43) Veröffentlichungstag der Anmeldung: 15.07.2009
(73) Patentinhaber: AGXX Intellectual Property Holding GmbH, 14129 Berlin (DE); Largentec Gmbh, 14129 Berlin (DE)
(72) Erfinder: LANDAU, Uwe, 14169 Berlin (DE); LISOWSKY, Thomas, 40789 Monheim (DE); ESSER, Karlheinz, 41199 Mönchengladbach (DE); MEHLER, Klaus-Dieter, 45470 Mülheim an der Ruhr (DE)
(74) Vertreter: Remus, Alvaro Johannes
(86) Internationale Anmeldenummer: PCT/EP2007/008564
(87) Internationale Veröffentlichungsnummer: WO 2008/046513

(56) Entgegenhaltungen:
- WO-A-01/43788
- WO-A-2004/011672
- US-A- 4 863 510

## Beschreibung

### Hintergrund der Erfindung

Die vorliegende Erfindung betrifft eine bioaktive Beschichtung, die zumindest Ruthenium oder Silber-Ruthenium-BimetallPartikel umfasst und auf eine Silber- oder silberhaltige Oberfläche aufgebracht oder mit einer Silber-Beschichtung in Kontakt ist , und ein Verfahren zur Beschichtung einer Vorrichtung sowie die Verwendung der bioaktiven Beschichtung.

Bereits 1869 wird durch Ravelin und 1893 durch von Nägeli die antibakterielle Wirkung von Silber in sehr niedrigen Dosierungen beschrieben. Die Wirksamkeit des Silbers hat auch heute an Aktualität nichts verloren (Landau, U.(2006): Die keimreduzierende Wirkung des Silbers in Hygiene, Medizin und Wasseraufbereitung: Die Oligodynamie des Silbers; Isensee-Verlag, Oldenburg, 2006-10-03).

Mikrobielle Kontaminationen verursachen weiterhin große Probleme und kommerzielle Verluste in allen Bereichen die mit Wasserqualität, wässrigen Lösungen und Hygiene in Zusammenhang stehen. Solche Bereiche findet man zum Beispiel in Krankenhäusern, Hygieneinstituten, in der Lebensmitteltechnologie, in der Produktion, in der Klimatechnik und auch im Haushalt.

Schon länger gibt es daher verschiedenste antimikrobielle Dekontaminationslösungen mit aggressiven chemischen Substanzen gegen Mikroorganismen, wie z.B. Formaldehyd, Alkohole, Phenole, Natriumazid, Natriummhypochlorit oder stark oxidierenden Agenzien, wie z.B. Hypochlorit, Bleichmittel oder mineralische Säure.

Die Nachteile dieser Lösungen und Methoden bestehen darin, dass die zur Dekontamination und Desinfektion verwendeten stark aggressiven Chemikalien und oxidierenden Agenzien ein hohes korrosives und toxisches Potenzial haben. Dadurch werden behandeltes Wasser und wässrige Lösungen für den Menschen üblicherweise ungenießbar und verwendete Geräte oder Oberflächen können korrosiv geschädigt werden.

Deshalb werden solche aggressiven chemischen Lösungen zur Waschung und Spülung von Geräten, Instrumenten und Arbeitsoberflächen üblicherweise in geschlossenen Kreisläufen verwendet.

Eine graduelle Verbesserung dieses Problems brachte die Anwendung der Silbertechnologie. Der oligodynamische Effekt des Silbers erlaubt die Entkeimung von Wasser oder wässrigen Lösungen in einer Qualität, die für den Menschen unbedenklich ist und Materialien und Oberflächen schont. Die Silbertechnologie wird daher auch für Trinkwasser bei dessen Herstellung, Aufbereitung und Qualitätssicherung verwendet.

Daher wird ständig an der Verbesserung der Effizienz der Silbertechnologie gearbeitet. Aus der WO 2005/023206 A2 und der DE 100 54 248 A1 sind beispielsweise neuere Verfahren bekannt, die Eigenschaften von Nanopartikeln nutzen, um über eine sehr große Oberfläche eine beschleunigte Abgabe von Silberionen zu erreichen.

Aus der WO 01/143788 A2 ist eine Vorrichtung bekannt, die mit einem Polymer beschichtet ist, das ein Kolloid umfasst. Das Kolloid entsteht dabei durch eine Reaktion eines ersten Salzes, das ein Silber- und/oder Ruthenium-Salz sein kann, mit einem zweiten Salz, das ein Ascorbat sein kann. Die WO 01/143788 A2 offenbart ferner ein Verfahren zur Herstellung einer kolloidhaltigen Polymerbeschichtung, die Silber, Ruthenium und Ascorbat umfasst, bei dem das Ascorbat dazu dient, mit Silber- und/oder Rutheni- um-Salzen ein Kolloid zu bilden, das dann eine erhöhte Kapazität für die Aufnahme antimikrobieller Ionen und eine Veränderung der Abgabekinetik der oligodynamischen Ionen bewirkt.

Das kommerzielle Interesse an Materialien und Methoden zur Erhaltung der Wasserqualität oder allgemein die Qualität wässriger Lösungen für den Menschen über die Silbertechnologie unterstreicht der bereits breite kommerzielle Vertrieb von entsprechenden Produkten unter den verschiedensten Markennamen.

Die Nachteile der bekannten Methoden zur Silbertechnologie sind ein stark verzögerter Einsatz der Wirkung nach einem Kontakt des Silbers mit Wasser und eine nur selektive antibakterielle Wirkung. So dauert es meist mehrere Stunden, häufig sogar wesentlich länger, bis nach einem Kontakt des Silbers mit kontaminiertem Wasser genügend Silberionen aus der Oberfläche freigesetzt werden, um eine ausreichende Abtötung der Mikroorganismen und eine Entkeimung des Wassers zu erreichen.

Die Silbertechnologie weist also zwei problematische Bereiche auf: 1. der zeitlich verzögerte Einsatz der keimabtötenden Wirkung und 2. das limitierte Wirkungsspektrum für die effiziente Dekontamination und Desinfektion von Wasser oder wässrigen Lösungen zur Abtötung oder Beseitigung von Mikroorganismen und problematischen Biomolekülen. Daher wird ständig nach verbesserten Methoden und Verfahren gesucht, um die Effizienz der Silbertechnologie zu erhöhen.

Die neuen Erkenntnisse der modernen Molekularbiologie und Gentechnologie zeigen weiterhin, dass neben Mikroorganismen auch schon Erbinformationen alleine, einzelne Gene oder sogar Teile davon, sowie bestimmte Proteine ausreichen, um Erkrankungen auszulösen oder unerwünschte genetische Veränderungen zu verursachen (Elhafi et al., 2004). Daher wäre eine effiziente Dekontamination von Oberflächen zur Abtötung oder Beseitigung von aktiven Biomolekülen ein zusätzlicher Sicherheitsgewinn in allen Bereichen der Wasserqualität und Hygiene.

Daher besteht in der Praxis ein Bedarf an verbesserten Materialien, Methoden und Verfahren zur effizienten und vor allem auch nicht-toxischen und nicht-korrosiven vollständigen Dekontamination und Desinfektion von Wasser oder wässrigen Lösungen zur Abtötung oder Beseitigung von Mikroorganismen und aktiven Biomolekülen, wie zum Beispiel DNA, RNA und Proteine.

### Zusammenfassung der Erfindung

Es ist daher Aufgabe der vorliegenden Erfindung, die Nachteile des Standes der Technik zu überwinden und neue Materialien und Verfahren zu entwickeln, die eine verbesserte und erweiterte Wirkung der Silbertechnologie erreichen.

Diese Aufgabe wird erfindungsgemäß dadurch gelöst, dass die Beschichtung zusätzlich mindestens ein Vitamin oder mindestens ein Derivat eines Vitamins und mindestens eine oberflächenaktive Substanz umfasst, wobei das Vitamin Ascorbinsäure ist.

Die Rutheniummoleküle auf der Silberoberfläche bzw. in direktem Kontakt mit Silbermolekülen dienen dabei zur schnelleren Freisetzung von Silberionen aber auch gleichzeitig als " Ankerpunkte" für die Bindung und Komplexierung von Ascorbinsäuremolekülen oder deren Derivaten.

Ferner wird die Aufgabe durch ein Verfahren zur Beschichtung einer Vorrichtung gelöst, nämlich durch das Aufbringen einer Ruthenium-Beschichtung auf eine Silber- oder silberhaltige Oberfläche der Vorrichtung, oder Aufbringen einer Silber-Beschichtung auf die Vorrichtung und anschließend Aufbringen einer Ruthenium-Beschichtung auf die Silber-Beschichtung, oder in Kontakt bringen einer Silber-Beschichtung und einer Ruthenium-Beschichtung, oder Aufbringen von Ruthenium-Silber-Partikeln auf die Vorrichtung; und Aufbringen von mindestens einem Vitamin oder mindestens einem Derivat eines Vitamins und mindestens einer oberflächenaktiven Substanz auf die Silber und Ruthenium umfassende Oberfläche der Vorrichtung, wobei das Vitamin Ascorbinsäure ist.

In der hier vorliegenden Erfindung wird der an sich bekannte oligodynamische Effekt des Silbers zur Keimreduktion erheblich verbessert und verstärkt durch die Kombination von Silber mit Ruthenium und Ascorbinsäure oder deren Derivaten, sowie einer oberflächenaktiven Substanz. Diese neuen bioaktiven Metalloberflächen führen zu einer schnelleren und effizienteren Abtötung von Mikroorganismen. Gleichzeitig verhindern diese neuen Metalloberflächen die Besiedelung mit Mikroorganismen und die Anheftung oder stabile Ablagerung von Biomolekülen, wie DNA, RNA oder Proteine. So erhält man eine sich selbst reinigende Oberfläche, die bei Kontakt mit Wasser oder wässrigen Lösungen sehr schnell und effizient Keimfreiheit und über längere Zeiträume erzeugt.

Bei der erfindungsgemäß mit zu verwendenden silberhaltigen Oberfläche oder Silberoberfläche kann es sich um die Oberfläche eines entsprechenden Kompakt- oder Vollmaterials handeln. Diese Oberfläche kann jedoch im Prinzip auch ein beliebig anderes Material (wie z.B. Kunststoff, Keramik, Glas etc.) mit einer aufgebrachten, dünnen, silberhaltigen Beschichtung umfassen, die zusammen mit der aufgebrachten äußeren Ruthenium-Beschichtung ein wirksames Ruthenium-Silber-Sandwich-System mit einer Silber- oder Silberlegierungs-Unterschicht oder - Unterlage und einer feuchtigkeits- oder nässedurchlässigen Ruthenium-Außenschicht, -Oberschicht oder -Deckschicht bildet.

Die Beschichtungen oder Be- schichtungssysteme, d.h. die Ruthenium-Beschichtung und eine ggf. erforderliche Silberunterschicht (i.d.R. mit einer Dicke von 2-10 µm) werden vorzugsweise galvanisch aufgebracht oder abgeschieden. Andere Beschichtungsverfahren, wie PVD-, CVD-, Sputter-, Sol-, Gel- und Reduktionsverfahren, sind ebenfalls hierfür geeignete Plattierverfahren.

Das Aufbringen der Ruthenium-Beschichtungen wird hierbei so gesteuert, dass die silberhaltige Oberfläche durch durchgehende, vorzugsweise fein ausgebildete, freie Flächen, Öffnungen, Poren, Risse, Zwischenräume oder dergleichen in der Ruthenium-Beschichtung im Feuchtigkeitskontakt mit der Umgebung steht oder in einen Feuchtigkeitskontakt mit der Umgebung gelangen kann und dadurch einen Feuchtigkeitskontakt zwischen Silber und dem Ruthenium gewährleistet ist. Wenn die Silberoberfläche mit Ruthenium-Clustern besetzt wird, kann der oligodynamische Effekt des Silbers in vorteilhafter Weise verstärkt werden. Die vorzugsweise clusterförmigen, porösen bzw. mikrorissigen, Ruthenium-Schichten in Kombination mit Silber erlauben eine sehr viel effizientere Abgabe von Silberionen in die Umgebung. Vorzugsweise wird Ruthenium in einer Dicke im Nanometer- oder Mikrometer-Bereich aufgebracht, wobei sich eine Dicke von maximal etwa 2 µm, insbesondere etwa 0,05 µm und minimal etwa 0,005-0,01 µm besonders bewährt hat.

Es können aber auch Ag-Ru-Partikel eingesetzt werden, bei denen Silber und Ruthenium in elektrisch leitendem Kontakt stehen und Feuchte oder Wasser beide Metalle benetzen. Die Silber-Ruthenium- Bimetallpartikel können im Mikro- oder Nanometer-Bereich, vorzugsweise im Nanometer-Bereich mit einer Größe von etwa <50 nm vorliegen. Silber- und Ruthenium-Partikel können auch als Einzelpartikel wirksam sein, wenn ein enger metallischer Kontakt zwischen beiden Partikelarten gegeben ist.

Nano- und Mikrometer große Me- tallpartikel können z.B. durch Mahl-, elektrochemische, chemisch reduktive, Kapillar-Elektrophorese-, Hydrothermal-Synthese-, PVD-, CVD- oder Sol-Gel-Verfahren hergestellt werden. Nanopartikel aus Ruthenium-Silber (Wu et al., 1990 II), Platin-Ruthenium (Schmidt et al., 1998), Ruthenium-Kupfer (Wu et al., 1990 I) und Ruthenium-Gold (Wu et al., 1990 II) werden nach Stand der Technik vorzugsweise für katalytische Zwecke hergestellt.

Das galvanische Abscheiden, insbesondere das Abscheiden der erfindungsgemäßen, vorzugsweise clusterförmigen, porösen bzw. mikrorissigen, Ruthenium-Schichten, kann durch die Auswahl eines geeigneten Elektrolyten, den Metallgehalt im Elektrolyten, die Elektrolyttemperatur, den pH-Wert des Elektrolyten, die Abscheidungsdauer oder Behandlungszeit und/oder über die Stromdichte bzw. Strommenge gezielt gesteuert werden. So sind insbesondere auch die Struktur und die Dimensionierung der erfindungsgemäßen Ruthenium-Cluster, (Mikro-)Poren und Mikrorisse in der Ruthenium-Schicht durch die gewählten galvanischen Abscheidungsbedingungen bestimmt, so dass die Dicke und die Struktur der Ruthenium-Schicht bedarfsgerecht einstellbar oder gestaltbar und an den jeweiligen Anwendungszweck optimal anpassbar ist. Beim galvanischen Abscheiden ist nicht nur eine einfache und bekannte herkömmliche Prozessführung verwendbar, wobei die einzelnen Schichten eines erfindungsgemäßen Ruthenium-Silber-Sandwich-Systems einfach nacheinander abgeschieden werden können, sondern es sind auch kommerziell verfügbare, bekannte und erprobte Elektrolytsysteme dafür einsetzbar.

Oberflächen werden vor dem Beschichten vorzugsweise zunächst gereinigt und einer Dekapierung und/oder einem Spülen unterzogen. Im Falle einer nicht elektrisch leitfähigen Vorrichtung muss die Oberfläche der Vorrichtung vor dem Aufbringen einer Silber- und Ruthenium-Beschichtung entsprechend der einem Galvaniker bekannten Verfahren vorbehandelt werden, um eine haftfeste Beschichtung mit Silber und Ruthenium zu ermöglichen.

Ein erster Hinweis auf die besondere synergistische Wechselwirkung zwischen Ruthenium und Ascorbinsäure liefert der Befund, dass Ruthenium sehr effizient in neuen Dekontaminations-lösungen mit Vitaminderivaten, Metallionen und Detergentien eingesetzt werden kann.

Es wurden neue Lösungen und Verfahren mit zwei- oder dreiwertigen Metallionen, Vitaminderivaten und Detergentien entwickelt, die die Nachteile des Standes der Technik für universelle Desinfektions- und Dekontaminations-Lösungen überwinden. und nicht mit aggressiven, korrosiven Chemikalien oder stark oxidierenden Agenzien arbeiten und außerdem die behandelten Substrate bei Raumtemperatur oder leicht erhöhten Temperaturen vollständig dekontaminieren.

Es ist bekannt, dass in physiologischen Mengen von mikromolaren Konzentrationen Antioxidantien in Kombination mit zweiwertigen Metallionen vereinzelt zu Schäden und partiellen Strangbrüchen an Nukleinsäuremolekülen führen können (Padayatty et al., 2003; Blokhina et al., 2003; Veal et al., 1991). Dies sind jedoch nur vereinzelte, isolierte Ergebnisse, die nur auf den jeweiligen Einzelfall anwendbar sind.

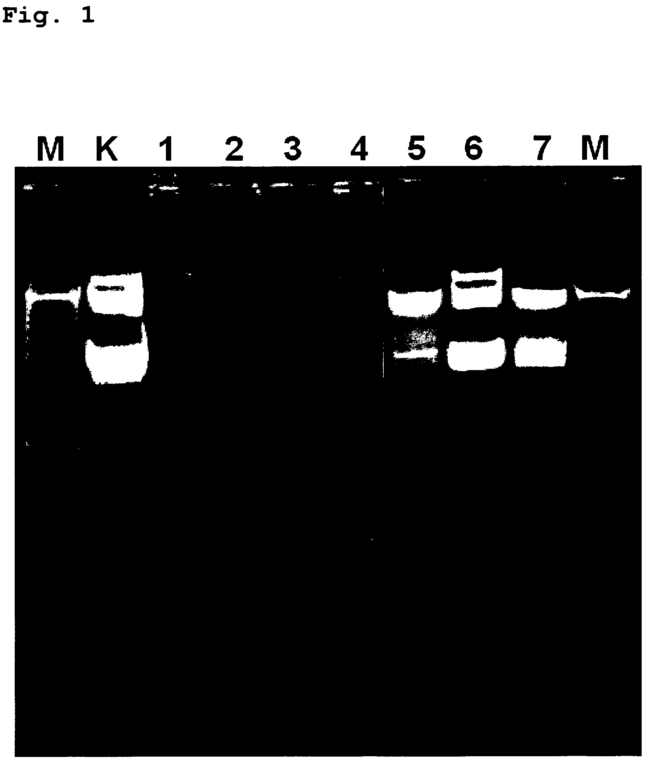

Das systematische Austesten neuer Kombinationen von Metallionen, Vitaminderivaten und Detergentien führte zur Entwicklung sehr effizienter, universeller, neuer Dekontaminations- und Desinfektionslösungen. Weiterführende neueste Versuche belegen, dass auch Ruthenium eine sehr effiziente synergistische Wirkung in diesem System hat (siehe Figur 1).

Überraschenderweise wurde dann aber zusätzlich neu gefunden, dass die Inkubation von neu entwickelten, mit Silber und Ruthenium beschichteten Metalloberflächen in Ascorbinsäurelösungen zu einer spontanen Bindung und Komplexierung der Ascorbinsäuremoleküle an den Rutheniummolekülen führt. Dadurch bildet sich ein nachhaltiges Depot aus Ascorbinsäuremolekülen an der Metalloberfläche. Möglich ist auch eine nebeneinander liegende Silber- und Ruthenium-Beschichtung, solange ein metallischer Kontakt gegeben ist. Denkbar ist auch eine poröse, mikrorissige Silberschicht über einer porösen Ruthenium-Schicht, sofern ein Kontakt zwischen beiden Metallen durch eine wässrige Lösung gegeben ist. Diese neu entwickelten Beschichtungsysteme aus Silber, Ruthenium, Ascorbinsäure und inerten Tensiden haben ganz neue überraschende Eigenschaften.

Die neuen Eigenschaften dieser Metalloberflächen führen zu einer sehr viel schnelleren und effizienteren Abtötung von Mikroorganismen als es nach dem Stand der Technik mit den bisherigen Materialien und Verfahren möglich ist.

Der Vergleich von verschiedenen Silberproben mit Silber/Ruthenium-Beschichtungen nach einer AscorbinsäureBehandlung in der Effizienz für die Keimabtötung macht dies deutlich.

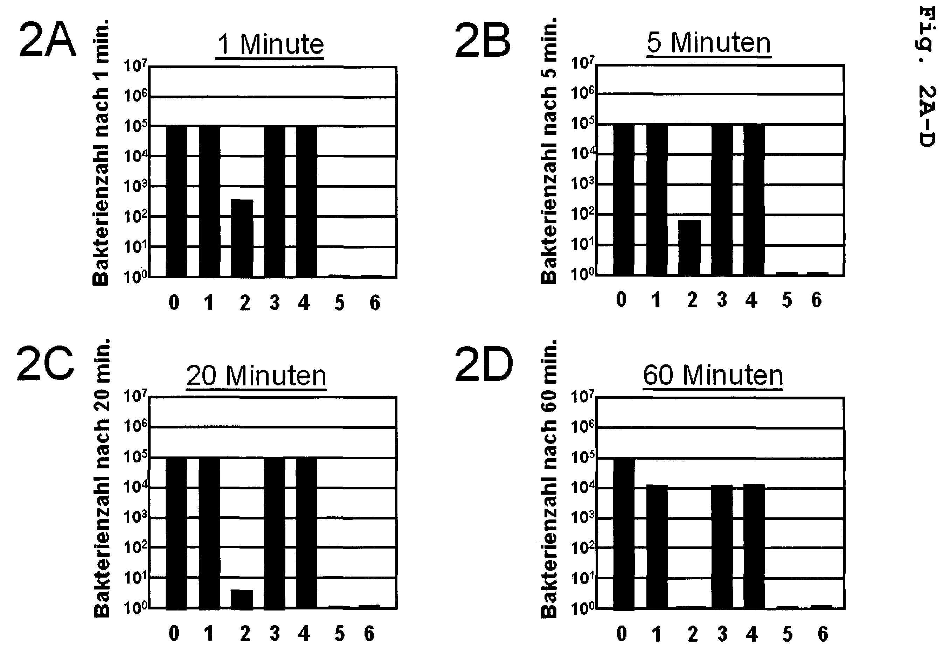

Runde Silberbleche von 1,3 cm Durchmesser wurden galvanisch mit entsprechenden mikroporösen Beschichtungen versehen und dann für 24 Stunden in 0,5 M wässriger Ascorbinsäurelösung inkubiert. Nach der Inkubation wurden die Bleche zweimal mit sterilem Wasser gewaschen. Ein so mit Ascorbinsäure behandeltes Silber/Ruthenium-Blech (Ag/Ru) zeigt eine sehr viel schnellere Keimabtötung als die Vergleichsproben. So sind Bakterien einer Testkultur schon nach 2 bis 20 Minuten drastisch in der Keimzahl reduziert oder sogar vollständig abgetötet (siehe die Figur 2A bis 2D). Nach der Behandlung mit Ascorbinsäure zeigen die verschiedenen Vergleichsproben mit reinem Silberblech (Ag), Silberblech mit Goldbeschichtung (Ag/Au) oder Silberblech mit Palladium- und Nickel-Beschichtung (Ag/Pd/Ni) erst nach ca. 60 Minuten Kontakt- und Einwirkungszeit die ersten signifikanten Erniedrigungen der bakteriellen Keimzahl, ohne eine vollständige Abtötung aller Keime in diesem Zeitraum zu erreichen.

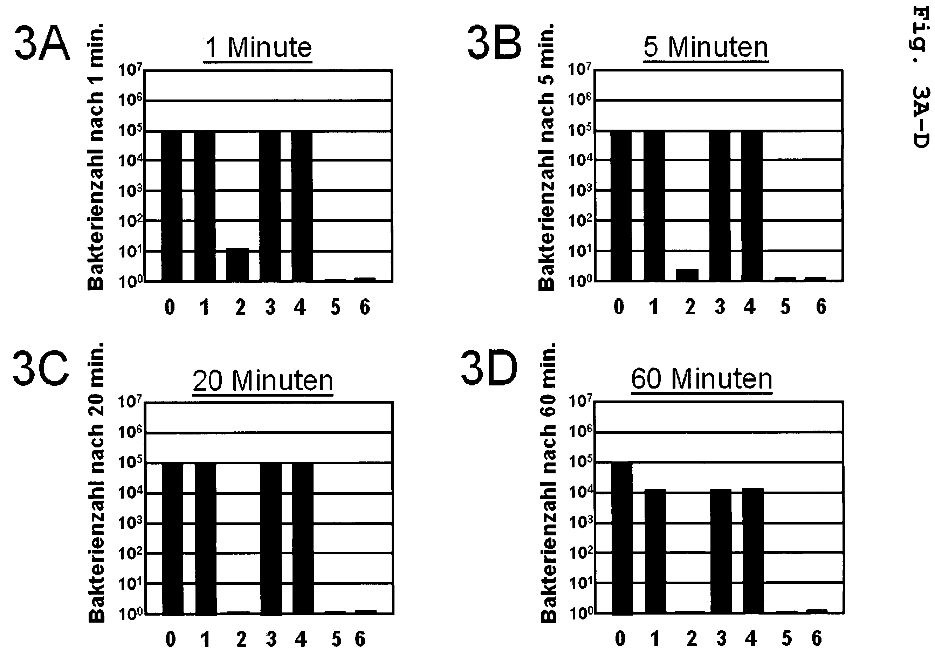

Auch ein mehrmaliges Waschen der mit Ascorbinsäure behandelten Silber-Ruthenium-Oberflächen mit sterilem Wasser bringt keine signifikante Reduzierung der erhöhten antibakteriellen Wirkung (siehe die Figuren 3A bis 3D). Dies zeigt, dass die Ascorbinsäuremoleküle auf der Oberfläche aus Silber und Ruthenium eine nachhaltige Bindung erfahren haben und ein Depot bilden. Weiterhin ergibt sich eine besondere synergistische Interaktion zwischen allen drei Komponenten.

Bei Bedarf kann das Depot an Ascorbinsäure durch einfaches Aufbringen oder sogar nur Abwischen mit einer wässrigen Ascorbinsäure-Lösung wieder aufgefüllt werden. Alternativ hierzu kann das Auffüllen des Depots auch durch eine erneute Inkubation in einer Ascorbinsäurelösung erfolgen.

Die Ergänzung des Depots aus Ascorbinsäure durch Aufbringung einer dünnen Schicht aus Ascorbinsäure und Detergentien ergibt den besonderen zusätzlichen Effekt, dass nun auch Nukleinsäuremoleküle bei Kontakt mit dieser Oberfläche schnell und vollständig abgebaut werden. Ein entscheidender neuer Punkt für die nachhaltige Depotbildung sind dabei die erfindungsgemäß bevorzugten, clusterförmigen, porösen bzw. mikrorissigen, Ruthenium-Silber-Schichten, die eine effiziente Depotbildung unterstützen.

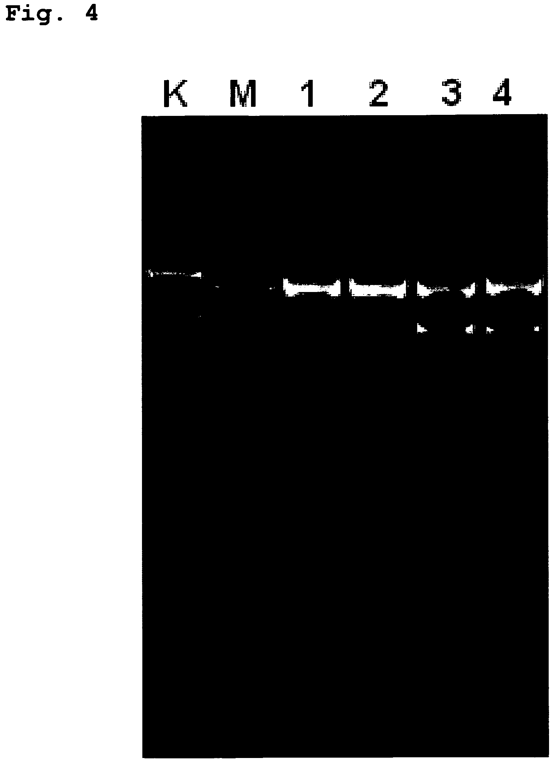

Ein Nachteil des Standes der Technik für die Silbertechnologie aber auch allgemein für die meisten bioaktiven Oberflächen ist eine fehlende Abbaufunktion für problematische Biomoleküle, wie DNA, RNA oder Proteine. Eigene Untersuchungen belegen, dass verschiedene Plastik- oder Metalloberflächen keine oder nur eine sehr begrenzte abbauende Wirkung auf DNA-Moleküle besitzen(siehe Figur 4).

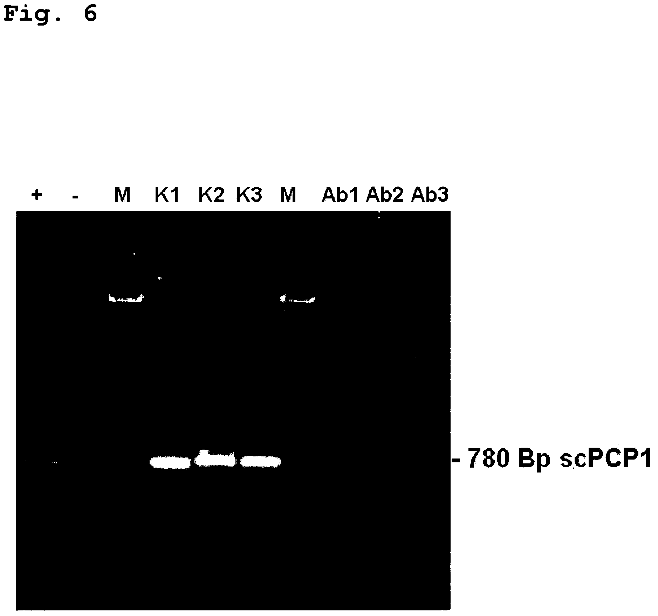

Die besondere synergistische Wirkung der neuen Ag/Ru-Beschichtungen in Kombination mit Ascorbinsäure und Detergentien zeigt sich bei Untersuchungen zur Stabilität von DNA-Molekülen auf so beschichteten Oberflächen. Werden definierte DNA-Proben als Kontamination auf diese Oberflächen aufgebracht so zeigt die Analyse durch Agarosegel und Elektrophorese einen schnellen, kompletten Abbau innerhalb von 30 Minuten bis 24 Stunden. Weitere Analysen mit der sehr empfindlichen PCR-Technologie zeigen, dass die aufgebrachten DNA-Moleküle schon nach 30 Minuten nicht mehr nachweisbar sind. siehe Fig. 6). Eine Vergleichsprobe auf einer Plastikoberfläche zeigt in diesem Zeitraum keinerlei DNA-Abbau. Damit haben die neuen bioaktiven Oberflächen die zusätzliche neue Eigenschaft einer sich selbstreinigenden Oberfläche für problematische Biomoleküle.

Ein weiterer Gegenstand der vorliegenden Erfindung ist die Verwendung der erfindungsgemäßen Beschichtung zur Herstellung und Erhaltung der Keimfreiheit von Wasser oder wässrigen Lösungen zur Sicherung der Hygiene und Wasserqualität.

Weitere vorteilhafte Ausführungsformen der vorliegenden Erfindung ergeben sich aus den Gegenständen der Unteransprüche.

Die effiziente Wirkung der neuen Metalloberflächen ist umso überraschender, als nachgewiesenermaßen die jeweiligen Einzelkomponenten keine vergleichbare effiziente Wirkung zeigen.

Erst die Beladung von Metalloberflächen oder Beschichtungen aus Silber und Ruthenium mit mindestens einem Vitamin oder dessen Derivaten und Detergen- tien führt zu einem synergistischen Effekt und zu einer beschleunigten, effizienteren Entkeimung wässriger Lösungen und zum
Abbau problematischer Biomoleküle an diesen Metall-Oberflächen oder Beschichtungen.

Die erfindungsgemäß herzustellenden Metalloberflächen oder Beschichtungen enthalten daher Silber, Ruthenium und Ascorbinsäure oder deren Derivate sowie mindestens eine oberflächenaktive Substanz.

Das vorzugsweise erfindungsgemäß mit zu verwendende Vitamin bzw. deren Salze oder sauren Derivate sind ein oder mehrere Verbindungen und/oder deren Salze ausgesucht aus der Gruppe der wasserlöslichen Vitamine mit den Eigenschaften von Antioxidanzien, nämlich Vitamin C. Es wird in Mengen von etwa 1 mM bis 1000 mM bezogen auf die Gesamtlösung mit verwendet, vorzugsweise in Mengen von etwa 10 mM bis 100 mM.

Eine zusätzliche Steigerung der Wirkung wird durch das Aufbringen von dünnen Schichten aus Ascorbinsäure oder deren Derivaten und zusätzlichen oberflächenaktiven Substanzen erreicht. Bei den erfindungsgemäß mit zu verwendenden oberflächenaktiven Substanzen handelt es sich um anionische, nichtionische, amphotere oder kationische inerte Tenside oder um geeignete Mischungen miteinander oder untereinander. Insbesondere können Alkylethersulfate, Alkyl- und/oder Arylsulfonate, Alkylsulfate, Amphotenside, Betaine, Alkylamidoalkylamine, alkylsubstituierte Aminosäuren, alkylsubstituierte Iminosäuren, acylierte Aminosäuren und Amphotensidkombinationen verwendet werden. Grundsätzlich sind alle inerten Tenside geeignet. Inert bedeutet, dass sie weder die synergistische Lösung noch das Versuchsergebnis beeinflussen. Erfindungsgemäß bevorzugt sind anionische und nichtionische Tenside.

Sie werden in Mengen von etwa 0.1 bis 10 Gew.-%, bezogen auf die Gesamtlösung verwendet, vorzugsweise in Mengen von etwa 0.2 bis 0.5 Gew.-%.

Auf die erfindungsgemäßen bioaktiven Oberflächen können zusätzlich auch noch zwei und/oder der dreiwertige Metallionen aufgebracht werden. Dies sind Ionen der Metalle der 4. Periode und/oder Nebengruppen I, II und VIII des periodischen Systems der Elemente. Sie werden in Form ihrer Salze mit organischen und/oder anorganische Säuren oder Basen eingesetzt. Erfindungsgemäß bevorzugt sind ein oder mehrere Verbindungen, ausgesucht aus der Nebengruppe VIII, insbesondere Eisen, Kobalt, Nickel, Kupfer oder Zink.

Sie werden in Mengen von etwa 1 mM bis 100 mM, bezogen auf die Gesamtlösung verwendet, vorzugsweise in Mengen von etwa 5 mM bis 10 mM.

Auf die erfindungsgemäßen bioaktiven Oberflächen können zusätzlich auch noch weitere übliche inerte Hilfs- und Zusatzstoffe aufgebracht werden, wie beispielsweise geeignete Puffersubstanzen zur Einstellung eines definierten pH-Wertes, wie Tris (Tris(hydroxymethyl)-aminomethan), MES (2-(Morpholino) ethansulfonsäure), HEPES (2-[4-(2-Hydroxyethyl)-1-piperazinyl] -ethansulfonsäure, MOPS (3-(N-Morpholino)propansulfonsäure), Carbonate und Derivate der Bernsteinsäure. Diese Puffersubstanzen werden in Mengen von etwa 1 mM bis 500 mM bezogen auf die Gesamtlösung verwendet.

Als Kontakt- und Einwirkzeit zwischen erfindungsgemäßen bioaktiven Metalloberflächen und Wasser oder wässrigen Lösungen sind etwa 5 bis 20 Minuten bei Raumtemperatur oder schwach erhöhter Temperatur im Allgemeinen ausreichend zur vollständigen Dekontamination und Desinfektion. Die angewandten Verfahren sind jedoch variierbar und können den jeweiligen Erfordernissen angepasst werden.

Die vorliegende Erfindung erlaubt die Entwicklung neuer bioaktiver Oberflächen für Hygiene-Anwendungen und zur Herstellung hoher Wasserqualität durch Beschichtungen aus Silber, Ruthenium und Ascorbinsäure oder deren Derivaten sowie einer oberflächenaktiven Substanz. Damit wird ein neuer Qualitätssprung in der Dekontamination und Desinfektion durch die Silbertechnologie erreicht, da hierdurch eine schnellere antimikrobielle Wirkung in Kombination mit einem nachhaltigen Langzeitschutz möglich ist. Gleichzeitig wird verhindert, dass sich aktive Biomoleküle, wie DNA, RNA oder Proteine, an der Oberfläche stabil anlagern.

Die Erfindung wird anhand der folgenden beispielhaften Figuren und Ausführungsbeispiele veranschaulicht.

### Kurze Beschreibung der Abbildungen

Dabei zeigt die Fig. 1 die besondere synergistische Wirkung zwischen Ruthenium und Ascorbinsäure am Beispiel der Degradation von DNA-Molekülen.
Die Fig. 2A-D zeigen die erhöhte antimikrobielle Wirkung der erfindungsgemäßen Beschichtungen aus Silber, Ruthenium und Ascorbinsäure im Vergleich mit anderen Silberproben nach dem Stand der Technik.
**Die** **Fig. 3A-D** zeigen den gleichen Test wie in Figur 2 nachdem die identischen Metallbleche aus Figur 2 erneut mit sterilem Wasser gewaschen und getrocknet wurden.
Fig. 4 zeigt eine Analyse zur Stabilität von DNA-Molekülen auf verschiedenen Oberflächen.
Fig. 5 zeigt eine Analyse des effizienten DNA-Abbaus auf den neuen Metalloberflächen mit einer speziellen Beschichtung.
**Fig. 6** zeigt eine PCR-Analytik von DNA-Proben nach verschiedenen Kontaktzeiten mit den erfindungsgemäß beschichteten neuen Metalloberflächen.

### Beispiele sowie Beschreibung verschiedener und bevorzugter Ausführungsformen der Erfindung

**Fig. 1** zeigt die besondere synergistische Wirkung zwischen Ruthenium und Ascorbinsäure am Beispiel der Degradation von DNA-Molekülen. Identische Aliquots von DNA-Plasmiden (YEp351) wurden für 2 Minuten mit den unten aufgeführten Lösungen zu Probe 1-7 behandelt. Anschließend wurden die DNA-Proben denaturiert und die einzelsträngigen DNA-Moleküle auf einem Agarosegel (1%) gelelektrophoretisch aufgetrennt. Nach der Färbung mit Ethidiumbromid erhält man die aufgeführten Bilder. Die Kontrolle zeigt die intakte Plasmid-DNA nach Behandlung mit sterilem Wasser. Bei der Einfügung von Strangbrüchen verkleinert sich das Molekulargewicht der betroffenen DNA-Moleküle. Dies kann im Gel durch einen Vergleich mit der Kontrolle und mit dem Molekulargewichtsmarker bestimmt werden. Es wurden jeweils 5 µg DNA in 5 µl sterilem Tris-Puffer (1 mM; pH 8.0) mit 5 µl der unten angegebenen Lösungen zu Probe 1-7 für 2 Minuten bei Raumtemperatur behandelt. Anschließend wurden die Proben mit 5 µl 100 mM Tris (pH 12) gemischt, mit Bromphenolblau-Marker versehen und für 5 Minuten bei 95°C denaturiert. Die denaturierten Proben wurden sofort auf 4°C abgekühlt und jeweils Aliquots mit 1 µg DNA pro Gelspur aufgetragen. Die DNA wurde nach der Gelelektrophorese im 1%-igen Agarosegel mit Ethidiumbromid angefärbt und fotografiert.

### Angaben zu den Proben:

M: DNA-Marker 1 kb Leiter; K: Kontrolle: DNA + 5 µl steriles H₂O; 1: 100 mM Ascorbinsäure + 10 mM FeCl₃; 2: 10 mM Ascorbinsäure + 1 mM FeCl₃; 3: 100 mM Ascorbinsäure + 10 mM RuCl₃; 4: 10 mM Ascorbinsäure + 1 mM RuCl₃; 5: 100 mM Ascorbinsäure + 10 mM AgNO₃ 6: 10 mM Benzoesäure; 7: 100 mM Ascorbinsäure

**Die** **Fig. 2A-D** zeigen die erhöhte antimikrobielle Wirkung der neuen Beschichtungen aus Silber, Ruthenium und Ascorbinsäure im Vergleich mit anderen Silberproben. Die Metallbleche (1,3cm Durchmesser) wurden in 0,5 M Ascorbinsäure-Lösung inkubiert, dann mit sterilem Wasser gewaschen und getrocknet. Nach dem Trocknen wurden die Proben in 1 ml sterilem Wasser enthaltend je 10⁵ Bakterien des Standardstammes *Escherichia coli* RRI geben. Die Anzahl lebender Bakterien wurde nach 1, 5, 20 und 60 Minuten Inkubation bestimmt und ist von 10⁵ bis 10⁰ angegeben.

Proben: 0: nur steriles H₂O; 1: reines Silberblech (Ag); 2: Silberblech mit Ruthenium (Ag/Ru), 3: Silberblech mit Gold Ag/Au); 4: Silberblech mit Palladium und Nickel(Ag/Pd/Ni); 5: 100 mM Ascorbinsäure, 10 mM FeCl₃, 6: 100 mM Ascorbinsäure, 10 mM RuCl₃, (5 + 6 je mit 0,3 % SDS und 0,2 % Tween 20).

**Die** **Fig. 3A-D** zeigen den gleichen Test wie in Figur 2 nachdem die identischen Metallbleche aus Figur 2 erneut mit sterilem Wasser gewaschen und getrocknet wurden. Nach dem Trocknen wurden die Proben erneut in 1 ml sterilem Wasser mit je 10⁵ Bakterien des Standardstammes *Escherichia coli* RRI geben. Die Anzahl lebender Bakterien wurde nach 1, 5, 20 und 60 Minuten Inkubation bestimmt und ist von 10⁵ bis 10⁰ angegeben.

Proben: 0: nur steriles H₂O; 1: reines Silberblech (Ag); 2: Silberblech mit Ruthenium (Ag/Ru), 3: Silberblech mit Gold (Ag/Au); 4: Silberblech mit Palladium und Nickel (Ag/Pd/Ni); 5: 100 mM Ascorbinsäure, 10 mM FeCl₃, 6: 100 mM Ascorbinsäure, 10 mM RuCl₃, (5 + 6 je mit 0,3 % SDS und 0,2 % Tween 20).

**Fig. 4** zeigt eine Analyse zur Stabilität von DNA-Molekülen auf verschiedenen Oberflächen. Es wurden jeweils 50 µl einer DNA-Lösung (25 ng/µl) auf die Oberflächen aufgetropft. Aliquots von jeweils 2 µl wurden nach 24 Stunden abgenommen und im analytischen Agarosegel untersucht. Die DNA wurde nach der Gelelektrophorese im 1%-ige Agarosegel mit Ethidiumbromid angefärbt und fotografiert. Als Kontroll-Oberfläche wurde steriles Plastikmaterial verwendet.

### Gelauftrag:

K: Kontrollprobe von Plastikoberfläche
M: Marker / 1 kb Leiter
1: DNA-Probe von Ag nach Ascorbinsäurebehandlung
2: DNA-Probe von Ag/Au nach Ascorbinsäurebehandlung
3: DNA-Probe von Ag/Ru nach Ascorbinsäurebehandlung
4: DNA-Probe von Pd/Ni nach Ascorbinsäurebehandlung

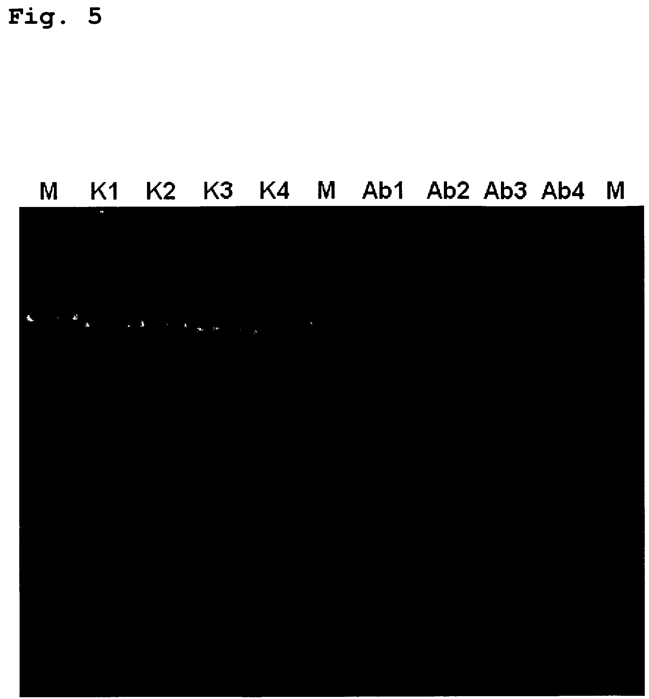

**Fig. 5** zeigt eine Analyse des effizienten DNA-Abbaus auf den neuen Metalloberflächen mit spezieller Beschichtung. Die Ag/Ru-Beschichtung wurde zusätzlich mit einer dünnen Schicht aus Ascorbinsäure, Metallionen und Detergentien versehen. Dafür wurde eine Lösung mit 100 mM Ascorbinsäure, 10 mM FeCl₃, 0.3 % SDS und 0,2 % Tween 20 durch kurzes Eintauchen, Abtropfen lassen und Antrocknung auf die Oberfläche gebracht. Anschließend wurden 50 µl einer DNA-Lösung (25 ng/µl) auf die Oberflächen aufgetropft. Aliquots von jeweils 2 µl wurden nach den angegebenen Zeiten abgenommen und die DNA wurde nach der Gelelektrophorese im 1%-igen Agarosegel mit Ethidiumbromid angefärbt und fotografiert. Als Kontroll-Oberfläche wurde steriles Plastikmaterial verwendet.

### Gelauftrag:

M: Marker / 1 kb Leiter
K1: Kontrollprobe der Plastikoberfläche nach 30 Minuten
K2: Kontrollprobe der Plastikoberfläche nach 1 Stunde
K3: Kontrollprobe der Plastikoberfläche nach 4 Stunden
K4: Kontrollprobe der Plastikoberfläche nach 24 Stunden
Ab1: DNA-Probe der beschichteten Oberfläche nach 30 Minuten
Ab2: DNA-Probe der beschichteten Oberfläche nach 1 Stunde
Ab3: DNA-Probe der beschichteten Oberfläche nach 4 Stunden
Ab4: DNA-Probe der beschichteten Oberfläche nach 24 Stunden

**Fig. 6** zeigt eine PCR-Analytik von DNA-Proben nach verschiedenen Kontaktzeiten mit den beschichteten neuen Metalloberflächen. Die Ag/Ru-Beschichtungen wurden zusätzlich mit einer dünnen Schicht aus Ascorbinsäure, Metallionen und Detergentien versehen. Dafür wurde eine Lösung mit 100 mM Ascorbinsäure, 10 mM FeCl₃, 0.3 % SDS und 0,2 % Tween 20 durch kurzes Eintauchen, Abtropfen lassen und Antrocknung auf die Oberfläche gebracht. Anschließend wurden 50 µl einer DNA-Lösung (0,1 ng/µl) auf die Oberfläche aufgetropft. Aliquots von jeweils 2 µl wurden nach den angegebenen Zeiten abgenommen und jeweils in einen 50 µl PCR-Reaktionsmix pipettiert. Der PCR-Reaktionsmix enthält Primerpaare für die Amplifikation der Test-DNA (scPCP1-Gen der Hefe). Die Kontrollen (+/-) zeigen an, ob die PCR-Reaktion erfolgreich verlaufen ist. Eine Bande von 780 Basenpaaren (Bp) der Test-DNA zeigt an, dass noch intakte DNA-Moleküle für dieses Gen vorhanden sind. Bei einer vollständigen Entfernung oder Zerstörung der Test-DNA sind keine amplifizierten DNA-Banden im Gel nachweisbar.
Die DNA wurde nach der Gelelektrophorese im 1%-ige Agarosegel mit Ethidiumbromid angefärbt und fotografiert. Als Kontroll-Oberfläche wurde steriles Plastikmaterial verwendet.

### Gelauftrag:

+: positive Kontrolle der PCR-Reaktion mit Test-DNA
-: negative Kontrolle der PCR-Reaktion ohne DNA
M: Marker / 1 kb Leiter
K1: Kontrollprobe von Plastikoberfläche nach 30 Minuten
K2: Kontrollprobe von Plastikoberfläche nach 1 Stunde
K3: Kontrollprobe von Plastikoberfläche nach 4 Stunden
Ab1: DNA-Probe von beschichteter Oberfläche nach 30 Minuten
Ab2: DNA-Probe von beschichteter Oberfläche nach 1 Stunde
Ab3: DNA-Probe von beschichteter Oberfläche nach 4 Stunden

### Literatur

Blokhina O., Virolainen E., Fagerstedt K.V. (2003): Antioxidants, Oxidative Damage and Oxygen Depriviation Stress: a Review. Annals Botany 91:179-194
Elhafi, G., Naylor, C.J., Savage, C.E. and Jones, R.C. (2004): Microwave or autoclave treatments destroy the infectivity of infectious bronchitis virus and avian pneumovirus but allow detection by reverse transcriptase-polymerase chain reaction. Avian Pathology 33, 3003-306
Padayatty S.J., Katz A., Wang Y., Eck P., Kwon O., Lee J.H., Chen S., Corpe C., Dutta A., Dutta S.K. and Levine M. (2003): Vitamin C as an antioxidant: evaluation of its role in disease prevention. J. Am. Coll. Nutr. 1, 18-35
Schmidt,T.J., Noeske,M., Gasteiger,H.A., Behm, R.J., Britz, P., Bönnemann, H. (1998): PtRu Alloy Colloids as Precursors for Fuel Cell catalysts. J. Electrochem. Soc. 145, 925
Veal J. M., Merchant K. & Rill R. L. (1991): The influence of reducing agent and 1,10-phenanthroline concentration on DNA cleavage by phenanthroline + copper. Nucl Acids Res Vol. 19, No. 12, 3383-3388
Wu, X., Gerstein, B.C., King, T.S. (1990) I: Characterization of Silica-Supported Cu Monometallic and Ru-Cu Bimetallic Catalysts by Hydrogen Chemisorption and NMR of Adsorbed Hydrogen. J. Catal.121, 271-293
Wu,X., Gerstein,B.C., King,T.S. (1990) II: Characterization of Silica-Supported Ru-Ag and Ru-Au Bimetallic Catalysts by Hydrogen Chemisorption and NMR of Adsorbed Hydrogen. J. of Catalysis 123,43-49

## Patentansprüche

1. Bioaktive Beschichtung, die zumindest Ruthenium oder Silber-Ruthenium-Bimetall-Partikel umfasst und auf eine Silber- oder silberhaltige Oberfläche aufgebracht oder mit einer Silber-Beschichtung in Kontakt ist, **dadurch gekennzeichnet, dass** die Beschichtung zusätzlich mindestens ein Vitamin oder mindestens ein Derivat eines Vitamins und mindestens eine oberflächenaktive Substanz umfasst, wobei das Vitamin Ascorbinsäure ist.

2. Bioaktive Beschichtung nach Anspruch 1, **dadurch gekennzeichnet, dass** diese derart ausgebildet ist, dass Silber-Ruthenium-Kontakte in einem Feuchtigkeitskontakt mit der Umgebung stehen.

3. Bioaktive Beschichtung nach Anspruch 1 oder 2, **dadurch gekennzeichnet, dass** diese mindestens ein zwei-oder dreiwertiges Metallion umfasst, wobei das Metallion ein Ion eines der Metalle der 4. Periode und/oder der Nebengruppen I, II, oder VIII des periodischen Systems der Elemente ist.

4. Bioaktive Beschichtung nach einem der Ansprüche 1 bis 3, **dadurch gekennzeichnet, dass** die oberflächenaktive Substanz mindestens eine Verbindung aus der Gruppe der anionischen, nichtionischen, amphoteren oder kationischen Tenside oder eine geeignete Mischung dieser Verbindungen ist.

5. Bioaktive Beschichtung nach einem der Ansprüche 1 bis 4, **dadurch gekennzeichnet, dass** die rutheniumhaltige Beschichtung eine Dicke von mindestens 5-10 nm und maximal 2µm aufweist.

6. Verfahren zur Beschichtung einer Vorrichtung durch:
Aufbringen einer Ruthenium-Beschichtung auf eine Silber- oder silberhaltige Oberfläche der Vorrichtung, oder Aufbringen einer Silber-Beschichtung auf die Vorrichtung und anschließend Aufbringen einer Ruthenium-Beschichtung auf die Silber-Beschichtung, oder in Kontakt bringen einer Silber-Beschichtung und einer Ruthenium-Beschichtung, oder Aufbringen von Ruthenium-Silber-Partikeln auf die Vorrichtung; und
Aufbringen von mindestens einem Vitamin oder mindestens einem Derivat eines Vitamins und mindestens einer oberflächenaktiven Substanz auf die Silber und Ruthenium umfassende Oberfläche der Vorrichtung, wobei das Vitamin Ascorbinsäure ist.

7. Verfahren nach Anspruch 6, **dadurch gekennzeichnet, dass** die Ruthenium-Beschichtung in einer Dicke von mindestens 5-10 nm und maximal 2µm aufgebracht wird.

8. Verfahren nach Anspruch 6 oder 7, **dadurch gekennzeichnet, dass** die Silber-Beschichtung in einer Dicke von 2-10 µm aufgebracht wird.

9. Verfahren nach einem der Ansprüche 6 bis 8, **dadurch gekennzeichnet, dass** die Silber-Ruthenium-Partikel als miteinander metallisch verbundene Bimetall-Partikel hergestellt werden.

10. Verfahren nach einem der Ansprüche 6 bis 8, **dadurch gekennzeichnet, dass** Ruthenium- und Silber-Partikel als reine Metall-Partikel hergestellt und zur Herstellung der Silber-Ruthenium-Partikel in einen engen metallischen Kontakt gebracht werden.

11. Verwendung der bioaktiven Beschichtung nach einem der Ansprüche 1 bis 5 zur Dekontaminierung oder Desinfektion von Wasser oder wässrigen Lösungen.

## Claims

1. Bioactive coating comprising at least ruthenium or silver-ruthenium bimetallic particles and being applied onto a silver or silver-containing surface or being in contact with a silver coating, **characterized in that** the coating additionally comprises at least one vitamin or at least one derivative of a vitamin and at least one surface-active substance, wherein the vitamin is ascorbic acid.

2. Bioactive coating according to claim 1, **characterized in that** it is designed in such a way that silver-ruthenium contacts are in moisture contact with the environment.

3. Bioactive coating according to claim 1 or 2, **characterized in that** it comprises at least one bi- or trivalent metal ion, wherein the metal ion is an ion of one of the metals of the 4^{th} period and/or subgroups I, II, or VIII of the periodic table of the elements.

4. Bioactive coating according to any one of claims 1 to 3, **characterized in that** the surface-active substance is at least one compound from the group of anionic, non-ionic, amphoteric, or cationic surfactants or a suitable mixture of these compounds.

5. Bioactive coating according to any one of claims 1 to 4, **characterized in that** the ruthenium-containing coating has a thickness of at least 5-10 nm and at most 2 µm.

6. Method for coating a device by:
applying a ruthenium coating to a silver or silver-containing surface of the device, or applying a silver coating to the device and subsequently applying a ruthenium coating to the silver coating, or bringing a silver coating in contact with a ruthenium coating, or applying ruthenium-silver particles to the device; and
applying at least one vitamin or at least one derivative of a vitamin and at least one surface-active substance to the device's surface comprising silver and ruthenium, wherein the vitamin is ascorbic acid.

7. Method according to claim 6, **characterized in that** the ruthenium coating is applied in a thickness of at least 5-10 nm and at most 2 µm.

8. Method according to claim 6 or 7, **characterized in that** the silver coating is applied in a thickness of 2-10 µm.

9. Method according to any one of claims 6 to 8, **characterized in that** the silver-ruthenium particles are produced as bimetallic particles that are metallically bonded to each other.

10. Method according to any one of claims 6 to 8, **characterized in that** ruthenium and silver particles are produced as pure metal particles and are brought into close metallic contact to produce the silver-ruthenium particles.

11. Use of the bioactive coating according to any one of claims 1 to 5 for decontamination or disinfection of water or aqueous solutions.

## Revendications

1. Revêtement bioactif qui comprend au moins du ruthénium ou des particules bimétalliques ruthénium-argent et qui est appliqué sur une surface en argent ou contenant de l'argent ou qui est en contact avec un revêtement d'argent, **caractérisé en ce que** le revêtement comprend en supplément au moins une vitamine ou au moins un dérivé d'une vitamine et au moins une substance tensio-active, la vitamine étant de l'acide ascorbique.

2. Revêtement bioactif selon la revendication 1, **caractérisé en ce que** celui-ci est conçu de telle sorte que des contacts ruthénium-argent soient en contact humide avec l'environnement.

3. Revêtement bioactif selon la revendication 1 ou la revendication 2, **caractérisé en ce que** celui-ci comprend un ion métallique bivalent ou trivalent, l'ion métallique étant un ion de l'un des métaux de la 4^{ème} période et/ou du 1^{er}, du 2^{ème} ou du 8^{ème} groupe secondaire du système périodique des éléments.

4. Revêtement bioactif selon l'une quelconque des revendications 1 à 3, **caractérisé en ce que** la substance tensio-active est au moins un composé du groupe des tensio-actifs anioniques, non ioniques, amphotères ou cationiques ou un mélange adapté desdits composés.

5. Revêtement bioactif selon l'une quelconque des revendications 1 à 4, **caractérisé en ce que** le revêtement contenant du ruthénium présente une épaisseur d'au moins 5 à 10 nm et d'un maximum de 2 µm.

6. Procédé destiné à revêtir un dispositif par :
application d'un revêtement au ruthénium sur une surface en argent ou contenant de l'argent du dispositif ou application d'un revêtement à l'argent sur le dispositif et application ensuite d'un revêtement au ruthénium sur le revêtement à l'argent ou mise en contact d'un revêtement à l'argent et d'un revêtement au ruthénium ou application de particules d'argent-ruthénium sur le dispositif ; et
application d'au moins une vitamine ou d'au moins un dérivé d'une vitamine et d'au moins une substance tensio-active sur la surface du dispositif comprenant de l'argent et du ruthénium, la vitamine étant de l'acide ascorbique.

7. Procédé selon la revendication 6, **caractérisé en ce qu'**on applique le revêtement au ruthénium sur une épaisseur d'au moins 5 à 10 nm et d'un maximum de 2 µm.

8. Procédé selon la revendication 6 ou la revendication 7, **caractérisé en ce qu'**on applique le revêtement à l'argent sur une épaisseur de 2 à 10 µm.

9. Procédé selon l'une quelconque des revendications 6 à 8, **caractérisé en ce qu'**on fabrique les particules ruthénium-argent en tant que particules bimétalliques métalliquement reliées entre elles.

10. Procédé selon l'une quelconque des revendications 6 à 8, **caractérisé en ce qu'**on fabrique des particules de ruthénium et d'argent en tant que pures particules métalliques et pour la fabrication des particules de ruthénium-argent, on les amène en contact métallique étroit.

11. Utilisation du revêtement bioactif selon l'une quelconque des revendications 1 à 5 pour décontaminer ou désinfecter de l'eau ou des solutions aqueuses.
